(19) **Europäisches Patentamt** / **European Patent Office** / **Office européen des brevets**

(11) **EP 4 620 385 A1**

(12) **EUROPEAN PATENT APPLICATION**
published in accordance with Art. 153(4) EPC

(43) Date of publication:
**24.09.2025   Bulletin 2025/39**

(21) Application number: **23891172.1**

(22) Date of filing: **26.09.2023**

(51) International Patent Classification (IPC):
**A61B 5/02** (2006.01)        **A61B 5/026** (2006.01)

(52) Cooperative Patent Classification (CPC):
**A61B 5/02; A61B 5/026**

(86) International application number:
**PCT/JP2023/034798**

(87) International publication number:
**WO 2024/106014 (23.05.2024 Gazette 2024/21)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority:   **17.11.2022   JP 2022184026**

(71) Applicant: **Sony Group Corporation**
**Tokyo 108-0075 (JP)**

(72) Inventors:
• **IKUTA, Tomoya**
**Tokyo 108-0075 (JP)**
• **KATSUHARA, Mao**
**Tokyo 108-0075 (JP)**

(74) Representative: **D Young & Co LLP**
**3 Noble Street**
**London EC2V 7BQ (GB)**

(54) **BIOMETRIC INFORMATION DETECTION DEVICE**

(57)     A biological information detection apparatus according to an embodiment of the present disclosure includes a housing, a light emitting device, a first optical member, a first light blocking member, and a light receiving device. The housing includes a detection surface. The light emitting device is configured to emit light toward the detection surface. The first optical member is provided between the light emitting device and the detection surface of the housing. The first light blocking member is configured to block light. The light receiving device is configured to detect light entering the detection surface. The light emitting device, the first light blocking member, and the light receiving device are disposed in this order in a first direction that is along the detection surface. The first optical member is configured to restrict a light traveling direction to prevent light emitted to an outside through the detection surface from traveling, in the first direction, beyond the first light blocking member into a direction in which the light receiving device is provided. The first optical member is configured to control the light traveling direction to cause the light emitted to the outside through the detection surface to travel while spreading, in the first direction, in a direction opposite to the direction in which the light receiving device is provided.

[ FIG. 5 ]

EP 4 620 385 A1

**Description**

Technical Field

The present disclosure relates to a biological information detection apparatus that detects biological information.

Background Art

There is a sensor that detects biological information such as a blood flow velocity or a pulse from a living body such as a human body. Examples of such a sensor include a photoplethysmography (PPG; photoplethysmography) sensor and a laser Doppler flowmetry (Laser Doppler Flowmetry) sensor. Such a sensor includes, for example, a light emitting device and a light receiving device. Light emitted from the light emitting device propagates inside the living body that is a detection target. The light receiving device detects light outputted from a surface of the living body. An amount of the light detected by the light receiving device changes in accordance with a change in volume of blood in the living body. For example, the photoplethysmography sensor is configured to detect biological information such as a pulse. Further, for example, the laser Doppler flowmetry sensor is configured to detect biological information such as a blood flow velocity or a pulse. For example, PTL 1 discloses a sensor configured to detect such biological information.

Citation List

Patent Literature

PTL 1: International Publication No. WO 2020/090883

Summary of the Invention

It is desired that a biological information detection apparatus have high detection accuracy, and it is hoped that detection accuracy is further improved.
It is desirable to provide a biological information detection apparatus that makes it possible to improve detection accuracy.
A biological information detection apparatus according to an embodiment of the present disclosure includes a housing, a light emitting device, a first optical member, a first light blocking member, and a light receiving device. The housing includes a detection surface. The light emitting device is configured to emit light toward the detection surface. The first optical member is provided between the light emitting device and the detection surface of the housing. The first light blocking member is configured to block light. The light receiving device is configured to detect light entering the detection surface. The light emitting device, the first light blocking member, and the light receiving device are disposed in this order in a first direction that is along the detection surface. The first optical member is configured to restrict a light traveling direction to prevent light emitted to an outside through the detection surface from traveling, in the first direction, beyond the first light blocking member into a direction in which the light receiving device is provided. The first optical member is configured to control the light traveling direction to cause the light emitted to the outside through the detection surface to travel while spreading, in the first direction, in a direction opposite to the direction in which the light receiving device is provided.
In the biological information detection apparatus according to the embodiment of the present disclosure, the light emitting device, the first light blocking member, and the light receiving device are disposed in this order in the first direction that is along the detection surface. A traveling direction of the light emitted by the light emitting device is controlled by the first optical member. The light emitted to the outside through the detection surface is so controlled as not to travel, in the first direction, beyond the first light blocking member into the direction in which the light receiving device is provided, and is so controlled as to travel while spreading, in the first direction, in the direction opposite to the direction in which the light receiving device is provided. Light entering the detection surface from a detection target is detected by the light receiving device.

Brief Description of the Drawings

[FIG. 1] FIG. 1 is a block diagram illustrating a configuration example of a biological information detection apparatus according to an embodiment of the present disclosure.
[FIG. 2] FIG. 2 is an explanatory diagram illustrating an example of a structure of the biological information detection apparatus illustrated in FIG. 1.

[FIG. 3A] FIG. 3A is an explanatory diagram illustrating a use example of the biological information detection apparatus illustrated in FIG. 1.

[FIG. 3B] FIG. 3B is an explanatory diagram illustrating another use example of the biological information detection apparatus illustrated in FIG. 1.

[FIG. 4] FIG. 4 is an explanatory diagram illustrating an operation example of the biological information detection apparatus illustrated in FIG. 1.

[FIG. 5] FIG. 5 is an explanatory diagram illustrating another operation example of the biological information detection apparatus illustrated in FIG. 1.

[FIG. 6] FIG. 6 is an explanatory diagram illustrating another operation example of the biological information detection apparatus illustrated in FIG. 1.

[FIG. 7] FIG. 7 is an explanatory diagram illustrating an example of a traveling direction of light emitted from the biological information detection apparatus illustrated in FIG. 1.

[FIG. 8] FIG. 8 is an explanatory diagram illustrating an example of a traveling direction of light entering the biological information detection apparatus illustrated in FIG. 1.

[FIG. 9] FIG. 9 is an explanatory diagram illustrating a design example of the biological information detection apparatus illustrated in FIG. 1.

[FIG. 10] FIG. 10 is an explanatory diagram illustrating a configuration example of a biological information detection apparatus according to a modification example.

[FIG. 11] FIG. 11 is an explanatory diagram illustrating a configuration example of a biological information detection apparatus according to another modification example.

[FIG. 12] FIG. 12 is an explanatory diagram illustrating a configuration example of a biological information detection apparatus according to another modification example.

[FIG. 13] FIG. 13 is an explanatory diagram illustrating a configuration example of a biological information detection apparatus according to a reference example.

[FIG. 14] FIG. 14 is an explanatory diagram illustrating a configuration example of a biological information detection apparatus according to another modification example.

[FIG. 15] FIG. 15 is an explanatory diagram illustrating a configuration example of a biological information detection apparatus according to another modification example.

[FIG. 16] FIG. 16 is an explanatory diagram illustrating an operation example of the biological information detection apparatus illustrated in FIG. 15.

[FIG. 17] FIG. 17 is an explanatory diagram illustrating a configuration example of a biological information detection apparatus according to another modification example.

[FIG. 18] FIG. 18 is an explanatory diagram illustrating a configuration example of a biological information detection apparatus according to another modification example.

[FIG. 19] FIG. 19 is an explanatory diagram illustrating a configuration example of a biological information detection apparatus according to another modification example.

[FIG. 20] FIG. 20 is an explanatory diagram illustrating a configuration example of a biological information detection apparatus according to another modification example.

[FIG. 21] FIG. 21 is an explanatory diagram illustrating a configuration example of a biological information detection apparatus according to another modification example.

[FIG. 22] FIG. 22 is an explanatory diagram illustrating a configuration example of a biological information detection apparatus according to another modification example.

[FIG. 23] FIG. 23 is an explanatory diagram illustrating a configuration example of a biological information detection apparatus according to another modification example.

[FIG. 24] FIG. 24 is a perspective diagram illustrating an outer appearance configuration of a watch to which the embodiment is applied.

Modes for Carrying Out the Invention

An embodiment of the present disclosure will be described below in detail with reference to the drawings. Note that the description will be given in the following order.

1. Embodiment
2. Application Example

<1. Embodiment>

[Configuration Example]

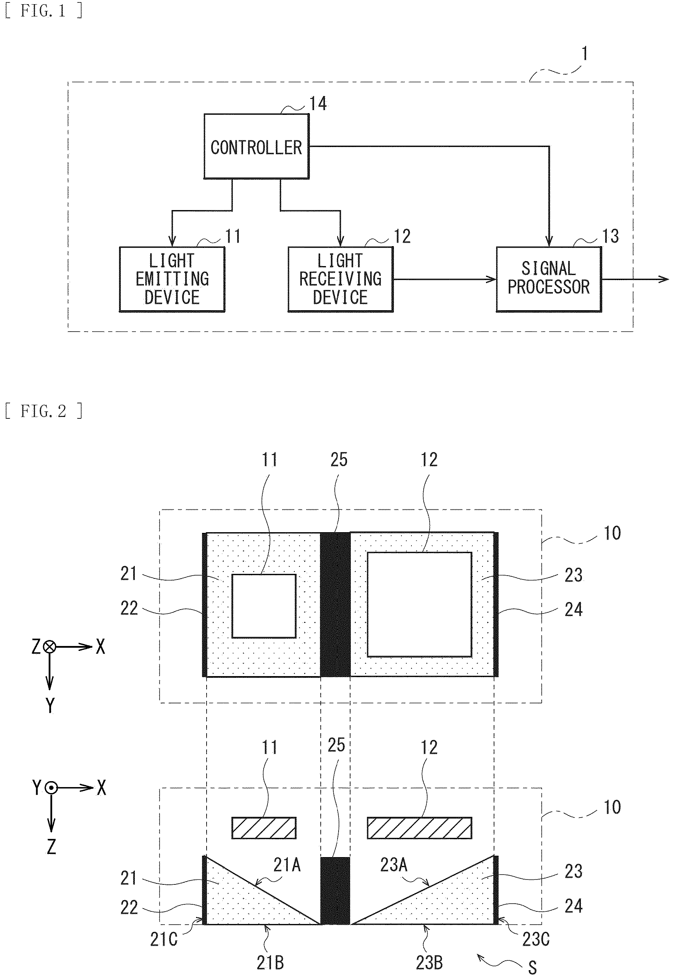

FIG. 1 illustrates a configuration example of a biological information detection apparatus (a biological information detection apparatus 1) according to an embodiment. The biological information detection apparatus 1 is to be provided in, for example, what is called a wearable device such as a wristband device. The biological information detection apparatus 1 is configured to, for example, detect biological information such as a blood flow velocity or a blood volume pulse. The biological information detection apparatus 1 includes a light emitting device 11, a light receiving device 12, a signal processor 13, and a controller 14.

The light emitting device 11 is configured to emit light. The light emitting device 11 includes, for example, an LED (Light Emitting Diode) or an LD (Laser Diode). For example, in a case where the LED is used, the biological information detection apparatus 1 serves as, for example, a photoplethysmography sensor, and is configured to detect a blood volume pulse. Alternatively, for example, in a case where the LD is used, the biological information detection apparatus 1 serves as, for example, a laser Doppler flowmetry sensor, and is configured to detect, for example, a blood flow velocity and a blood volume pulse. A wavelength of the light may be a wavelength in a visible range or a wavelength in a near infrared range or an infrared range.

The light receiving device 12 is configured to detect light and to generate a detection signal corresponding to an amount of received light. The light receiving device 12 includes, for example, a PD (Photo Diode).

The signal processor 13 is configured to perform predetermined signal processing on the basis of the detection signal supplied from the light receiving device 12 and to output a result of the processing.

The controller 14 is configured to control an overall operation of the biological information detection apparatus 1 by controlling respective operations of the light emitting device 11, the light receiving device 12, and the signal processor 13.

FIG. 2 illustrates an example of disposing each member in the biological information detection apparatus 1. The biological information detection apparatus 1 is housed in a housing 10. The biological information detection apparatus 1 emits light toward a living body through a detection surface S of the housing 10, and detects light entering from the living body through the detection surface S. The biological information detection apparatus 1 includes a prism 21, a light blocking film 22, a prism 23, a light blocking film 24, and a light blocking wall 25. The light emitting device 11 and the light receiving device 12 are disposed side by side in an X direction.

The prism 21 is configured to control a traveling direction of the light emitted from the light emitting device 11. The prism 21 is provided between the light emitting device 11 and the detection surface S in a Z direction. The prism 21 includes, for example, glass or plastic. The prism 21 has a triangular prism shape extending in a Y direction, and has a cross-sectional shape in an XZ plane that is a right triangle in this example. The prism 21 includes an inclined surface 21A and surfaces 21B and 21C. The inclined surface 21A is a surface inclined from the detection surface S and faces the light emitting device 11. The surface 21B is a surface along the detection surface S of the biological information detection apparatus 1. The surface 21C is a surface on a side opposite to a side on which the light receiving device 12 is provided in the X direction. The prism 21 is disposed in such an orientation that a thickness in the Z direction decreases toward the light receiving device 12 in the X direction.

With this configuration, the prism 21 so restricts a light traveling direction as to prevent light emitted to an outside through the surface 21B from traveling, in the X direction, beyond the light blocking wall 25 into a direction (a right direction in FIG. 2) in which the light receiving device 12 is provided, and so controls the light traveling direction as to cause the light emitted to the outside through the surface 21B to travel while spreading, in the X direction, in a direction (a left direction in FIG. 2) opposite to the direction in which the light receiving device 12 is provided. In addition, the prism 21 is configured to so control the light traveling direction as to cause the light emitted to the outside through the surface 21B to travel while spreading in the Y direction.

The light blocking film 22 is provided on the surface 21C of the prism 21 in the X direction. The light blocking film 22 is configured to block, for example, light arriving at the surface 21C, out of light traveling inside the prism 21.

The prism 23 is configured to control a traveling direction of light entering the detection surface S of the biological information detection apparatus 1 from the living body. The prism 23 is provided between the light receiving device 12 and the detection surface S. The prism 23 includes, for example, glass or plastic. The prism 23 has a triangular prism shape extending in the Y direction, and has a cross-sectional shape in the XZ plane that is a right triangle in this example. The prism 23 includes an inclined surface 23A and surfaces 23B and 23C. The inclined surface 23A is a surface inclined from the detection surface S and faces the light receiving device 12. The surface 23B is a surface along the detection surface S of the biological information detection apparatus 1. The surface 23C is a surface on a side opposite to a side on which the light emitting device 11 is provided in the X direction. The prism 23 is disposed in such an orientation that a thickness in the Z direction decreases toward the light emitting device 11 in the X direction.

With this configuration, the prism 23 is configured to so restrict a light traveling direction as to prevent light that has entered the surface 23B from a direction in which the light emitting device 11 is provided and has an incident angle of a predetermined angle or greater, out of light entering the surface 23B from the outside, from traveling, in the X direction, toward the light receiving device 12.

The light blocking film 24 is provided on the surface 23C of the prism 23 in the X direction. The light blocking film 24 is configured to block, for example, light arriving at the surface 23C, out of light that travels inside the prism 23.

The light blocking wall 25 is provided between the prism 21 and the prism 23 in the X direction. The light blocking wall 25 is configured to block, for example, light directly arriving at the light blocking wall 25 from the light emitting device 11 and light arriving at the light blocking wall 25 from the outside.

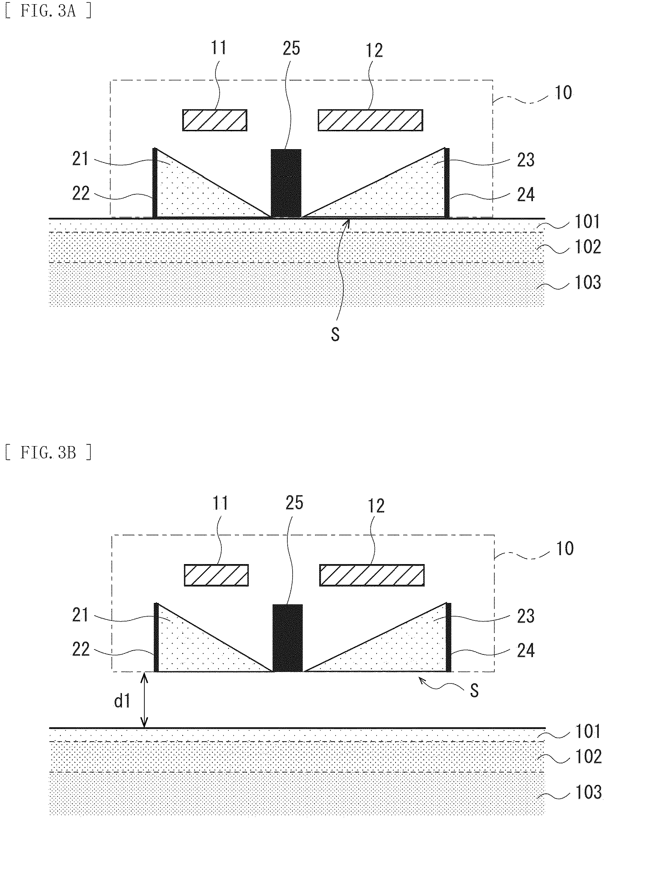

FIGs. 3A and 3B each illustrate a use example of the biological information detection apparatus 1. FIG. 3A illustrates a state in which the detection surface S of the biological information detection apparatus 1 is in contact with a surface of skin of a human body. FIG. 3B illustrates a state in which the detection surface S of the biological information detection apparatus 1 is spaced away from the surface of the skin. In this example, a distance between the detection surface S of the biological information detection apparatus 1 and the surface of the skin is a distance d1.

The skin of the human body includes an epidermis 101, a dermis 102, and a subcutaneous tissue 103. The epidermis 101, the dermis 102, and the subcutaneous tissue 103 are provided in this order from the surface of the skin. For example, the light emitted from the light emitting device 11 enters the surface of the skin via the prism 21. Thereafter, the light propagates inside the human body, and the light having propagated inside the human body enters the light receiving device 12 via the prism 23. Because the epidermis 101 has no blood vessel, an amount of light that propagates through the epidermis 101 hardly changes over time. In contrast, because the dermis 102 and the subcutaneous tissue 103 have blood vessels, an amount of light that propagates through the dermis 102 and the subcutaneous tissue 103 changes in accordance with a change in volume of blood flowing through the blood vessels. As a result, light detected by the light receiving device 12 includes a light component (a DC component) that hardly changes over time, and a light component (an AC component) that changes in accordance with a change in volume of blood. The biological information detection apparatus 1 is configured to detect the biological information on the basis of the AC component.

The biological information detection apparatus 1 is desirably in contact with the surface of the skin as illustrated in FIG. 3A. However, the biological information detection apparatus 1 can be slightly spaced away from the surface of the skin as illustrated in FIG. 3B.

For example, in a case where the prism 21 is not provided and the biological information detection apparatus 1 is in contact with the surface of the skin as illustrated in FIG. 3A, the light emitted from the light emitting device 11 enters an epidermis portion, of the epidermis 101, that is in contact with the surface 21B of the prism 21. However, when the biological information detection apparatus 1 becomes slightly spaced away from the surface of the skin as illustrated in FIG. 3B, the light emitted from the light emitting device 11 can enter a wider epidermis portion, of the epidermis 101, below the light emitting device 11 and, in addition, can also enter an epidermis portion below the light receiving device 12. In this case, the light receiving device 12 detects light with an increased amount of the DC component, which can result in deterioration in detection accuracy. In the biological information detection apparatus 1 according to the present embodiment, the prism 21 is provided, and the prism 21 so restricts the light traveling direction as to prevent the light emitted to the outside through the detection surface S from traveling, in the X direction, beyond the light blocking wall 25 into the direction (a right direction in FIG. 3B) in which the light receiving device 12 is provided. This prevents the light emitted from the light emitting device 11 from easily entering the epidermis portion below the light receiving device 12, suppressing an increase in the DC component included in the light to be detected by the light receiving device 12. In addition, in the biological information detection apparatus 1, the prism 21 so controls the light traveling direction as to cause the light emitted to the outside through the detection surface S to travel while spreading in directions (a left direction in FIG. 3B and the Y direction in FIG. 2) other than the direction in which the light receiving device 12 is provided. This increases an area in which light enters the human body and thus allows more blood to be irradiated with the light, which makes it possible to increase the AC component included in the light to be detected by the light receiving device 12. As a result, the biological information detection apparatus 1 makes it possible to improve detection accuracy.

Here, the housing 10 corresponds to a specific example of a "housing" in one embodiment of the present disclosure. The light emitting device 11 corresponds to a specific example of a "light emitting device" in one embodiment of the present disclosure. The prism 21 corresponds to a specific example of a "first optical member" in one embodiment of the present disclosure. The inclined surface 21A corresponds to a specific example of a "first inclined surface" in one embodiment of the present disclosure. The light blocking wall 25 corresponds to a specific example of a "first light blocking member" in one embodiment of the present disclosure. The prism 23 corresponds to a specific example of a "second optical member" in one embodiment of the present disclosure. The inclined surface 23A corresponds to a specific example of a "second inclined surface" in one embodiment of the present disclosure. The X direction corresponds to a specific example of a "first direction" in one embodiment of the present disclosure. The Y direction corresponds to a specific example of a "second direction" in one embodiment of the present disclosure.

[Operation and Workings]

Next, a description is given of an operation and workings of the biological information detection apparatus 1 of the present embodiment.

(Overview of Overall Operation)

First, an overview of an overall operation of the biological information detection apparatus 1 will be given with reference to FIGs. 1 and 2. The light emitting device 11 emits light. The prism 21 controls the traveling direction of the light emitted from the light emitting device 11. The prism 23 controls the traveling direction of the light entering the detection surface S of the biological information detection apparatus 1 from the outside. The light blocking films 22 and 24 and the light blocking wall 25 block light. The light receiving device 12 detects light and generates the detection signal corresponding to the amount of the received light. The signal processor 13 performs the predetermined signal processing on the basis of the detection signal supplied from the light receiving device 12. The controller 14 controls the overall operation of the biological information detection apparatus 1 by controlling the respective operations of the light emitting device 11, the light receiving device 12, and the signal processor 13.

(Detailed Operation)

Next, the operation of the biological information detection apparatus 1 will be described in detail. First, a description is given of a case where the detection surface S of the biological information detection apparatus 1 is in contact with the surface of the skin, and then, a description is given of a case where the detection surface S of the biological information detection apparatus 1 is spaced away from the surface of the skin.

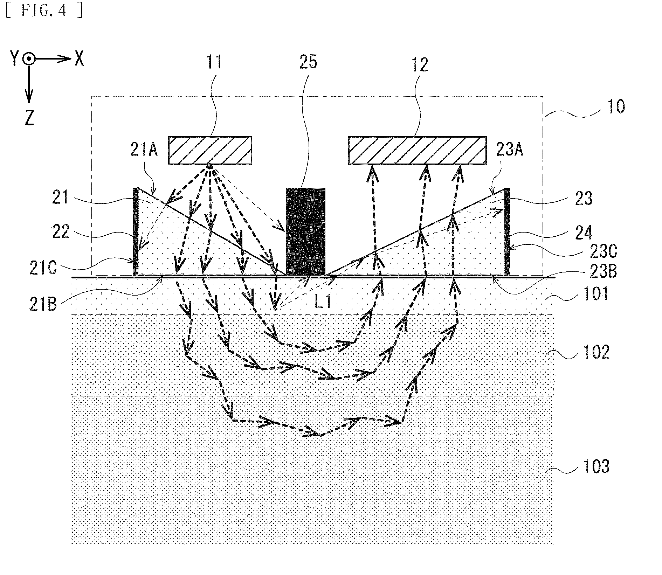

FIG. 4 illustrates an operation example of the biological information detection apparatus 1 in the case where the detection surface S of the biological information detection apparatus 1 is in contact with the surface of the skin. In FIG. 4, dashed-line arrows each indicate the light traveling direction.
The light emitted from the light emitting device 11 travels while spreading toward the inclined surface 21A of the prism 21. Out of this light, light arriving at the light blocking wall 25 is blocked by the light blocking wall 25. Light arriving at the inclined surface 21A of the prism 21 is, for example, refracted at an interface of the inclined surface 21A, and the refracted light travels inside the prism 21. Out of this light, light arriving at the light blocking film 22 is blocked by the light blocking film 22. Light arriving at the surface 21B of the prism 21 is, for example, refracted at an interface of the surface 21B, and the refracted light enters the human body. In this example, because the detection surface S of the biological information detection apparatus 1 is in contact with the surface of the skin, light from the prism 21 enters an epidermis portion in contact with the surface 21B of the prism 21. Further, this light propagates inside the human body.
An amount of light that propagates through the dermis 102 and the subcutaneous tissue 103 out of the light that propagates inside the human body changes in accordance with a change in volume of blood. A portion of this light arrives at, for example, the surface 23B of the prism 23. The light arriving at the surface 23B of the prism 23 is, for example, refracted at an interface of the surface 23B, and the refracted light travels inside the prism 23. Light arriving at the inclined surface 23A of the prism 23 is, for example, refracted at an interface of the inclined surface 23A, and the refracted light travels toward, for example, the light receiving device 12.
In contrast, an amount of light that propagates through the epidermis 101 out of the light that propagates inside the human body hardly changes over time. A portion of this light directly arrives at the light blocking wall 25 from an epidermis portion below the light emitting device 11, and is blocked by the light blocking wall 25. Further, a portion (light L1) of this light enters the prism 23 from the epidermis portion below the light emitting device 11. The light L1 enters the surface 23B of the prism 23 at a large incident angle, is refracted at the interface of the surface 23B, and the refracted light travels inside the prism 23 and arrives at the inclined surface 23A of the prism 23. An incident angle of the light is large also at the inclined surface 23A. Therefore, the light is totally reflected at the interface of the inclined surface 23A. The totally reflected light travels inside the prism 23, and the traveling light arrives at the light blocking film 24 and is blocked by the light blocking film 24.
In this manner, the light propagating through the epidermis 101 may be blocked by the light blocking wall 25 or may be totally reflected at the interface of the inclined surface 23A of the prism 23. This allows the light to be detected by the light receiving device 12 to include an increased amount of the light component (the AC component) that changes in accordance with a change in volume of blood, and reduces an amount of the light component (the DC component) that hardly changes over time. Accordingly, it is possible for the biological information detection apparatus 1 to detect the biological information at high accuracy on the basis of such an AC component.

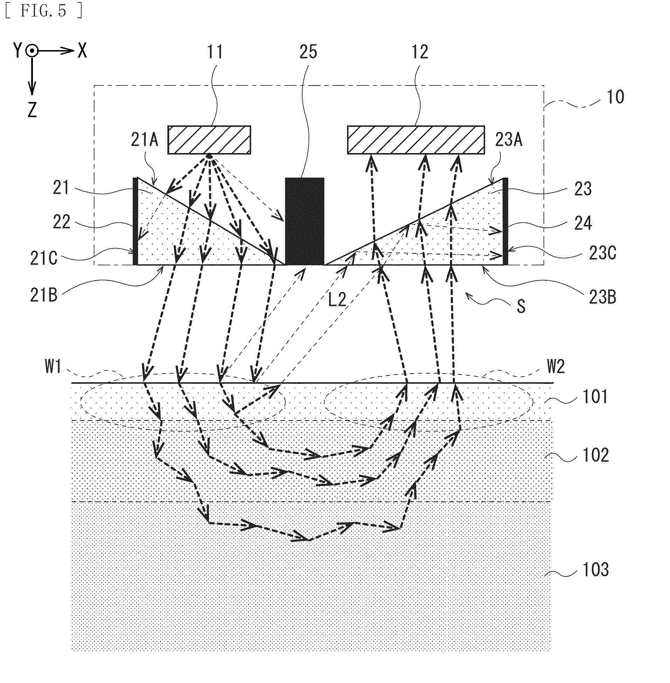

FIG. 5 illustrates an operation example of the biological information detection apparatus 1 in the case where the detection surface S of the biological information detection apparatus 1 is spaced away from the surface of the skin.
As with the case where the detection surface S is in contact with the surface of the skin (FIG. 4), the light emitted from the light emitting device 11 travels while spreading toward the inclined surface 21A of the prism 21. Light arriving at the inclined surface 21A of the prism 21 is, for example, refracted at the interface of the inclined surface 21A, and the refracted light travels inside the prism 21. Light arriving at the surface 21B of the prism 21 is, for example, refracted at the interface of the surface 21B, and the refracted light travels through a space between the biological information detection apparatus 1 and the surface of the skin.

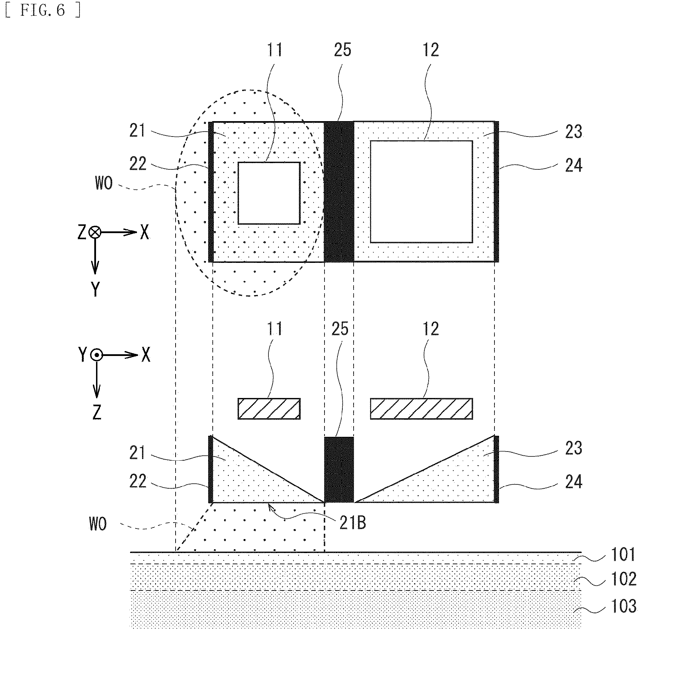

FIG. 6 illustrates an example of the traveling direction of the light emitted to the outside through the surface 21B. In FIG. 6, a region WO indicates a region in which the light emitted to the outside through the surface 21B travels. The prism 21 so restricts the light traveling direction as to prevent the light emitted to the outside through the surface 21B from traveling, in the X direction, beyond the light blocking wall 25 into the direction (a right direction in FIG. 6) in which the light receiving device 12 is provided, and so controls the light traveling direction as to cause the light emitted to the outside through the surface 21B to travel while spreading, in the X direction, in the direction (a left direction in FIG. 6) opposite to the direction in which the light receiving device 12 is provided. In addition, the prism 21 so controls the light traveling direction as to cause the light emitted to the outside through the surface 21B to travel while spreading in the Y direction.

Further, light that has traveled through the space between the biological information detection apparatus 1 and the surface of the skin enters the human body. The light that has entered the human body propagates inside the human body.

An amount of light that propagates through the dermis 102 and the subcutaneous tissue 103 out of the light that propagates inside the human body changes in accordance with a change in volume of blood. A portion of this light arrives at, for example, the surface 23B of the prism 23 (FIG. 5). Because the prism 21 so controls the light traveling direction as to cause the light emitted to the outside through the surface 21B of the prism 21 to travel while spreading in the directions (the left direction and the Y direction in FIG. 6) other than the direction in which the light receiving device 12 is provided, the area in which the light enters the human body is large. This allows more blood to be irradiated with the light, which increases the light component that arrives at the surface 23B of the prism 23 and changes in accordance with a change in volume of blood. The light arriving at the surface 23B of the prism 23 is, for example, refracted at the interface of the surface 23B, and the refracted light travels inside the prism 23. Light arriving at the inclined surface 23A of the prism 23 is, for example, refracted at the interface of the inclined surface 23A, and the refracted light travels toward, for example, the light receiving device 12.

An amount of light that propagates through the epidermis 101 out of the light that propagates inside the human body hardly changes over time. A portion of this light directly arrives at the light blocking wall 25 from an epidermis portion W1 below the light emitting device 11, and is blocked by the light blocking wall 25. Further, a portion (light L2) of this light enters the prism 23 from the epidermis portion W1 below the light emitting device 11. The light L2 enters the surface 23B of the prism 23 at a slightly large incident angle. In this example, because the detection surface S of the biological information detection apparatus 1 is spaced away from the surface of the skin, the incident angle at which the light L2 enters the surface 23B of the prism 23 tends to be smaller than that in the case illustrate in FIG. 4. However, it is possible to maintain the incident angle to be large to some extent, because the prism 21 so restricts the light traveling direction as to prevent the light emitted to the outside through the surface 21B from traveling, in the X direction, beyond the light blocking wall 25 into the direction (the right direction in FIG. 6) in which the light receiving device 12 is provided. In such a manner, the light entering the surface 23B of the prism 23 is refracted at the interface of the surface 23B, and the refracted light travels inside the prism 23 and arrives at the inclined surface 23A of the prism 23. Because an incident angle of the light at the interface of the inclined surface 23A is large, the light is totally reflected at the interface of the inclined surface 23A. The totally reflected light travels inside the prism 23, and the traveled light arrives at the light blocking film 24 and is blocked by the light blocking film 24.

In this manner, the light propagating through the epidermis 101 may be blocked by the light blocking wall 25 or may be totally reflected at the interface of the inclined surface 23A of the prism 23. This allows the light to be detected by the light receiving device 12 to include an increased amount of the light component (the AC component) that changes in accordance with a change in volume of blood, and reduces an amount of the light component (the DC component) that hardly changes over time. Accordingly, it is possible for the biological information detection apparatus 1 to detect the biological information at high accuracy on the basis of such an AC component.

(Regarding Prism 21)

The prism 21 so restricts the light traveling direction as to prevent the light emitted to the outside through the surface 21B from traveling, in the X direction, beyond the light blocking wall 25 into the direction (the right direction in FIG. 6) in which the light receiving device 12 is provided, and so controls the light traveling direction as to cause the light emitted to the outside through the surface 21B to travel while spreading in the directions (the left direction and the Y direction in FIG. 6) other than the direction in which the light receiving device 12 is provided. These workings of the prism 21 will be described below in detail.

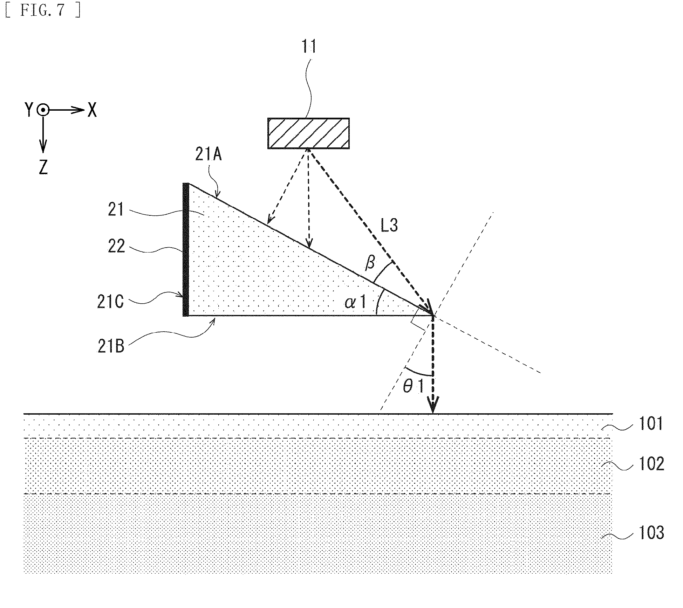

FIG. 7 illustrates an example of the workings of the prism 21. FIG. 7 illustrates the light emitting device 11, the prism 21, the light blocking film 22, and the skin (the epidermis 101, the dermis 102, and the subcutaneous tissue 103) of the human body. In this example, an angle formed by the inclined surface 21A and the surface 21B in the prism 21 is an angle $\alpha 1$.

The light emitted from the light emitting device 11 travels while spreading toward the inclined surface 21A of the prism 21. A portion (light L3) of this light travels toward an end part of the prism 21, of the inclined surface 21A of the prism 21, that is closest to the light receiving device 12. An angle formed by a traveling direction of the light L3 and the inclined surface 21A is an angle β. The light L3 is refracted at the interface of the inclined surface 21A, and the refracted light travels toward the human body. An angle formed by a traveling direction of this light and a normal line to the inclined surface 21A is an angle θ1. It is possible to express a relationship between the angle β and the angle θ1 by the following expression using a refractive index n1 of air and a refractive index n2 of the prism 21.

$$n1 \times \sin (90°-β) = n2 \times \sin θ1$$

On the basis of this expression, the following expression is obtainable.

$$n1 \times \cos β = n2 \times \sin θ1$$

On the basis of this expression, Expression (EQ1) is obtainable.

$$n1/n2 \times \cos β = \sin θ1 \ ...... \ (EQ1)$$

As illustrated in FIG. 6, the prism 21 so restricts the light traveling direction as to prevent the light emitted to the outside through the surface 21B from traveling, in the X direction, beyond the light blocking wall 25 into the direction (the right direction in FIG. 6) in which the light receiving device 12 is provided. This working is achieved in a case where the following expression is satisfied.

$$θ1 \leq α1 \ ...... \ (EQ2)$$

Accordingly, the following expression is obtainable from Expression (EQ1) and Expression (EQ2).

$$n1/n2 \times \cos β \leq \sin α1 \ ...... \ (EQ3)$$

For example, allowing the prism 21 to have a configuration satisfying Expression (EQ3) makes it possible for the prism 21 to so restrict the light traveling direction as to prevent the light emitted to the outside through the surface 21B from traveling, in the X direction, beyond the light blocking wall 25 into the direction (the right direction in FIG. 6) in which the light receiving device 12 is provided. The light emitted to the outside through the surface 21B is thus prevented from easily entering an epidermis portion W2 below the light receiving device 12. This reduces an amount of light that propagates through the epidermis portion W2, of the epidermis 101, below the light receiving device 12, and thus suppresses an increase in the DC component included in the light to be detected by the light receiving device 12. As a result, the biological information detection apparatus 1 makes it possible to improve the detection accuracy.

In addition, as illustrated in FIG. 7, the prism 21 is disposed in such an orientation that the thickness in the Z direction decreases toward the light receiving device 12 in the X direction. This allows the prism 21 to so control the light traveling direction as to cause the light emitted to the outside through the surface 21B to travel while spreading, in the X direction, in the direction (a left direction in FIG. 7) opposite to the direction in which the light receiving device 12 is provided. This also allows the prism 21 to so control the light traveling direction as to cause the light emitted to the outside through the surface 21B to travel while spreading also in the Y direction. Accordingly, the light emitted to the outside through the surface 21B travels while spreading toward the epidermis portion W1 below the light emitting device 11, and enters the epidermis portion W1. This allows more blood to be irradiated with light, and thus makes it possible to increase the AC component included in the light to be detected by the light receiving device 12. As a result, the biological information detection apparatus 1 makes it possible to improve the detection accuracy.

(Regarding Prism 23)

The prism 23 so restricts the light traveling direction as to prevent light that has entered the surface 23B from the direction in which the light emitting device 11 is provided and has the incident angle of the predetermined angle or greater, out of light entering the surface 23B from the outside, from traveling, in the X direction, toward the light receiving device 12. This working of the prism 23 will be described below in detail.

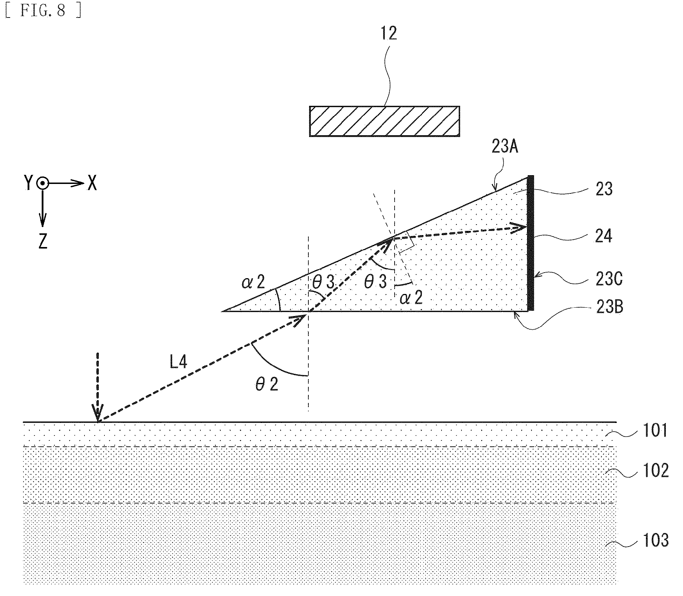

FIG. 8 illustrates an example of the working of the prism 23. FIG. 8 illustrates the light receiving device 12, the prism 23, the light blocking film 24, and the skin (the epidermis 101, the dermis 102, and the subcutaneous tissue 103) of the human body. In this example, an angle formed by the inclined surface 23A and the surface 23B in the prism 23 is an angle α2.

A portion (light L4) of the light from the epidermis portion W1 below the light emitting device 11 travels toward the surface 23B of the prism 23. An angle formed by a traveling direction of the light L4 and a normal line to the surface 23B is an angle θ2. The light L4 is refracted at the interface of the surface 23B of the prism 23, and the refracted light travels toward the inclined surface 23A of the prism 23. This refraction angle is an angle θ3. Further, this light is reflected by total reflection at the interface of the inclined surface 23A of the prism 23, and the reflected light travels toward the light blocking film 24. It is possible to express a relationship between the angle θ2 and the angle θ3 by the following expression using the refractive index n1 of the air and a refractive index n2 of the prism 23.

$$n1 \times \sin \theta2 = n2 \times \sin \theta3$$

On the basis of this expression, the following expression is obtainable.

$$\sin \theta3 = n1 / n2 \times \sin \theta2$$

On the basis of this expression, the following expression is obtainable.

$$\theta3 = \arcsin (n1/n2 \times \sin \theta2) \ ...... \ (EQ4)$$

The prism 23 so controls the light traveling direction as to cause the light that has entered the surface 23B from the direction in which the light emitting device 11 is provided, out of the light entering the surface 23B from the outside, to be totally reflected at the interface of the inclined surface 23A and travel, in the X direction, toward the light blocking film 24. This working is achieved when the following expression is satisfied.

$$\sin (\theta3 + \alpha2) \geq n1/n2 \ ...... \ (EQ5)$$

Accordingly, the following expression is obtainable from Expression (EQ4) and Expression (EQ5).

$$\sin (\arcsin (n1/n2 \times \sin \theta2) + \alpha2) \geq n1/n2 \ ...... \ (EQ6)$$

For example, allowing the prism 23 to have a configuration satisfying Expression (EQ6) makes it possible for the prism 23 to so control the light traveling direction as to cause the light that has entered the surface 23B from the direction in which the light emitting device 11 is provided and has the incident angle of the predetermined angle (the angle θ2) or greater, out of the light entering the surface 23B from the outside, to be totally reflected at the interface of the inclined surface 23A of the prism 23. This light is thus prevented from entering the light receiving device 12. This suppresses an increase in the DC component included in the light to be detected by the light receiving device 12. As a result, the biological information detection apparatus 1 makes it possible to improve the detection accuracy.

As described above, the biological information detection apparatus 1 is provided with the prisms 21 and 23. This makes it possible, in the case where the detection surface S is spaced away from the surface of the skin, to increase the AC component included in the light to be detected by the light receiving device 12 and to suppress an increase in the DC component. It is thus possible for the biological information detection apparatus 1 to efficiently detect a change in volume of blood in the living body, and therefore to improve the detection accuracy.

(Design Example)

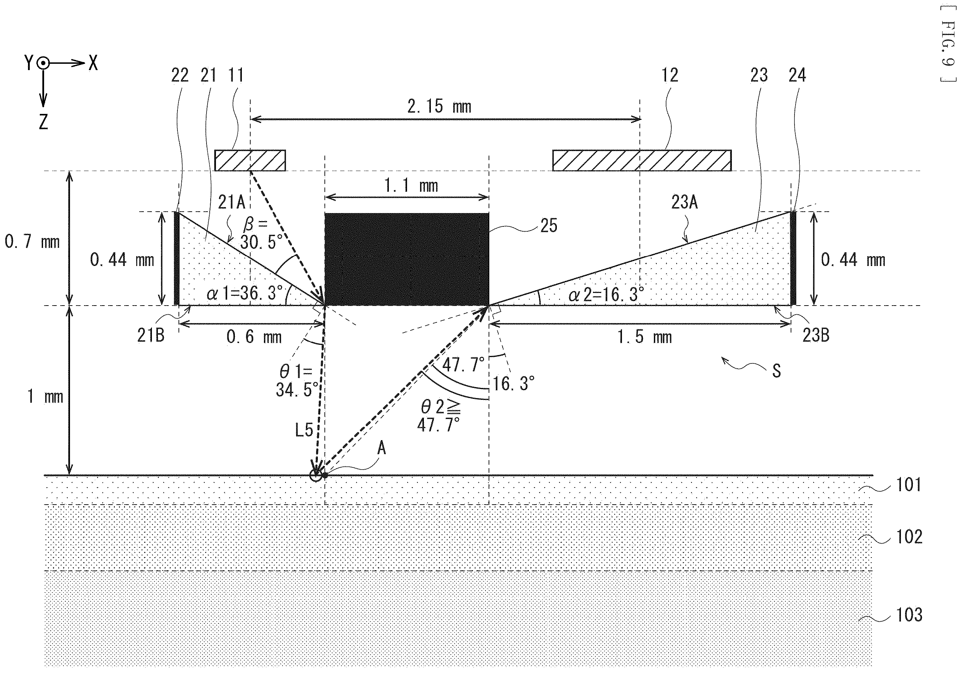

FIG. 9 illustrates a design example of the biological information detection apparatus 1.

In this example, a distance between a center position of the light emitting device 11 and a center position of the light receiving device 12 in the X direction is 2.15 mm. In this example, the center position of the light emitting device 11 is the same as a center position of the prism 21 in the X direction, and the center position of the light receiving device 12 is the same as a center position of the prism 23 in the X direction. Note that this is non-limiting, and the center position of the light emitting device 11 may be deviated from the center position of the prism 21, and the center position of the light receiving device 12 may be deviated from the center position of the prism 23.

In this example, a distance between a light emitting surface of the light emitting device 11 and the detection surface S (the surface 21B of the prism 21) of the biological information detection apparatus 1 is 0.7 mm, and a distance between a light receiving surface of the light receiving device 12 and the detection surface S (the surface 23B of the prism 23) of the biological information detection apparatus 1 is 0.7 mm. The refractive index n1 of the air is 1, and the refractive index n2 of each of the prisms 21 and 23 is 1.52 in this example.

In this case, the prism 21 has a width of 0.6 mm in the X direction, and a height of 0.44 mm in the Z direction. The angle $\alpha 1$ is 36.3°, the angle $\beta$ is 30.5°, and the angle $\theta 1$ is 34.5°. Accordingly, the angle $\theta 1$ (34.5°) is smaller than or equal to the angle $\alpha 1$ (36.3°), which allows the condition of Expression (EQ2) to be satisfied, and thus allows the condition of Expression (EQ3) to be satisfied. This allows the prism 21 to so restrict the light traveling direction as to prevent the light emitted to the outside through the surface 21B from traveling, in the X direction, beyond the light blocking wall 25 into the direction (a right direction in FIG. 9) in which the light receiving device 12 is provided. Light L5 refracted at the end part, of the prism 21, on the light receiving device 12 side travels toward the human body. A position, in the X direction, at which the light L5 enters the human body is on a side (a left side in FIG. 9) opposite to the side on which the light receiving device 12 is provided, relative to a position (a position A) of the end part of the prism 21. Therefore, the prism 23 is desirably so designed as to prevent light that has propagated through an epidermis portion on the left side relative to the position A and has outputted from this epidermis portion, from entering the light receiving device 12.

In this example, the prism 23 has a width of 1.5 mm in the X direction, and has a height of 0.44 mm in the Z direction. The angle $\alpha 2$ is 16.3°. The light blocking wall 25 has a width of 1.1 mm in the X direction. In this example, the distance between the detection surface S of the biological information detection apparatus 1 and the surface of the skin of the human body is 1 mm. In this example, assumed is a case where light enters the end part, of the prism 23, on the light emitting device 11 side, from the epidermis portion on the left side relative to the position A. In this case, the angle $\theta 2$ is greater than or equal to 47.7°. Therefore, the condition of Expression (EQ6) is satisfied. This allows the prism 23 to so control the light traveling direction as to cause the light that has entered the surface 23B from the direction in which the light emitting device 11 is provided and has the incident angle of the predetermined angle (47.7° in this example) or greater, out of the light entering the surface 23B from the outside, to be totally reflected at the interface of the inclined surface 23A. In such a manner, it is possible for the prism 23 to so restrict the light traveling direction as to prevent the foregoing light from traveling toward the light receiving device 12.

Note that the design example of the biological information detection apparatus 1 has been described, referring to FIG. 9, with specific values, but these values are merely examples and are non-limiting.

As described above, the biological information detection apparatus 1 includes the housing 10, the light emitting device 11, a first optical member (the prism 21), the light blocking wall 25, and the light receiving device 12. The housing 10 includes the detection surface S. The light emitting device 11 is configured to emit light toward the detection surface S. The first optical member (the prism 21) is provided between the light emitting device 11 and the detection surface S of the housing 10. The light blocking wall 25 is configured to block light. The light receiving device 12 is configured to detect light entering the detection surface S. The light emitting device 11, the light blocking wall 25, and the light receiving device 12 are disposed in this order in a first direction (the X direction) that is along the detection surface S. The first optical member (the prism 21) is configured to restrict the light traveling direction to prevent the light emitted to the outside through the detection surface S from traveling, in the first direction (the X direction), beyond the light blocking wall 25 into the direction in which the light receiving device 12 is provided, and is configured to control the light traveling direction to cause the light emitted to the outside through the detection surface S to travel while spreading, in the first direction (the X direction), in the direction opposite to the direction in which the light receiving device 12 is provided. For example, in the case where the detection surface S of the biological information detection apparatus 1 is spaced away from the surface of the skin, this prevents the light emitted to the outside through the surface 21B from easily entering the epidermis portion W2 below the light receiving device 12. This reduces an amount of light propagating through the epidermis portion W2, of the epidermis 101, below the light receiving device 12, and thus makes it possible to suppress an increase in the DC component included in the light to be detected by the light receiving device 12. Further, the light emitted to the outside through the surface 21B travels while spreading toward the epidermis portion W1 below the light emitting device 11, and enters the epidermis portion W1. This allows a lot of blood to be irradiated with light, which makes it possible to increase the AC component included in the light to be detected by the light receiving device 12. As a result, the biological information detection apparatus 1 makes it possible to improve the detection accuracy.

That is, for example, in the case where the prism 21 is not provided, if the biological information detection apparatus becomes spaced away from the surface of the skin, the light emitted from the light emitting device 11 can enter not only the epidermis portion W1, of the epidermis 101, below the light emitting device 11, but also the epidermis portion W2, of the epidermis 101, below the light receiving device 12. In this case, the DC component in the light to be detected by the light receiving device 12 can increase, which can deteriorate the detection accuracy. In contrast, in the biological information detection apparatus 1 according to the present embodiment, the light traveling direction is so restricted that the light emitted to the outside through the detection surface S is prevented from traveling, in the X direction, beyond the light blocking wall 25 into the direction in which the light receiving device 12 is provided. This prevents the light emitted to the outside through the surface 21B from easily entering the epidermis portion W2 below the light receiving device 12. Accordingly, in the case where the biological information detection apparatus 1 is spaced away from the surface of the skin, it is possible to suppress an increase in the DC component included in the light to be detected by the light receiving device 12, and to thus improve the detection accuracy.

In addition, for example, in a case where a light collecting member such as a convex lens is provided, instead of the prism 21, between the light emitting device 11 and the detection surface S of the housing 10, the light emitted from the light emitting device 11 enters a narrow epidermis portion below the light emitting device 11. Therefore, less blood is irradiated with light. This can reduce the AC component included in the light to be detected by the light receiving device 12, which can deteriorate the detection accuracy. In contrast, in the biological information detection apparatus 1 according to the present embodiment, the light traveling direction is so controlled as to cause the light emitted to the outside through the detection surface S to travel while spreading, in the X direction, in the direction opposite to the direction in which the light receiving device 12 is provided. Accordingly, the light emitted from the light emitting device 11 enters a wide epidermis portion below the light receiving device 12, which allows a lot of blood to be irradiated with light. This makes it possible to increase the AC component included in the light to be detected by the light receiving device 12, and thus makes it possible to improve the detection accuracy.

In addition, in the biological information detection apparatus 1, the first optical member (the prism 21) is configured to control the light traveling direction to cause the light emitted to the outside through the detection surface S to travel while spreading along the detection surface S, in a second direction (the Y direction) intersecting with the first direction (the X direction). Accordingly, the light emitted to the outside through the surface 21B travels while spreading toward the epidermis portion W1 below the light emitting device 11, and enters the epidermis portion W1. This allows a lot of blood to be irradiated with light, and thus makes it possible to increase the AC component included in the light to be detected by the light receiving device 12. As a result, the biological information detection apparatus 1 makes it possible to improve the detection accuracy.

In addition, the biological information detection apparatus 1 further includes a second optical member (the prism 23) provided between the light receiving device 12 and the detection surface S of the housing 10. The second optical member (the prism 23) is configured to restrict the light traveling direction to prevent light that has entered the detection surface S from the direction in which the light emitting device 11 is provided and has an incident angle of the predetermined angle or greater, out of light entering the detection surface S from the outside, from traveling, in the first direction (the X direction), toward the light receiving device 12. This prevents the light propagating through the epidermis portion W1 below the light emitting device 11 from easily entering the light receiving device 12. Accordingly, it is possible to suppress an increase in the DC component included in the light to be detected by the light receiving device 12. As a result, the biological information detection apparatus 1 makes it possible to improve the detection accuracy.

[Effects]

As described above, in the present embodiment, provided are a housing, a light emitting device, a first optical member, a light blocking wall, and a light receiving device. The housing includes a detection surface. The light emitting device is configured to emit light toward the detection surface. The first optical member is provided between the light emitting device and the detection surface of the housing. The light blocking wall is configured to block light. The light receiving device is configured to detect light entering the detection surface. The light emitting device, the light blocking wall, and the light receiving device are disposed in this order in a first direction that is along the detection surface. The first optical member is configured to restrict a light traveling direction to prevent light emitted to an outside through the detection surface from traveling, in the first direction, beyond the light blocking wall into a direction in which the light receiving device is provided. The first optical member is configured to control the light traveling direction to cause the light emitted to the outside through the detection surface to travel while spreading, in the first direction, in a direction opposite to the direction in which the light receiving device is provided. This makes it possible to improve the detection accuracy.

In the present embodiment, the first optical member is configured to control the light traveling direction to cause the light emitted to the outside through the detection surface to travel while spreading along the detection surface, in a second direction intersecting with the first direction. This makes it possible to improve the detection accuracy.

In the present embodiment, a second optical member is further provided. The second optical member is provided between the light receiving device and the detection surface of the housing. The second optical member is configured to restrict the light traveling direction to prevent light that has entered the detection surface from a direction in which the light emitting device is provided and has an incident angle of a predetermined angle or greater, out of light entering the detection surface from the outside, from traveling, in the first direction, toward the light receiving device. This makes it possible to improve the detection accuracy.

[Modification Example 1]

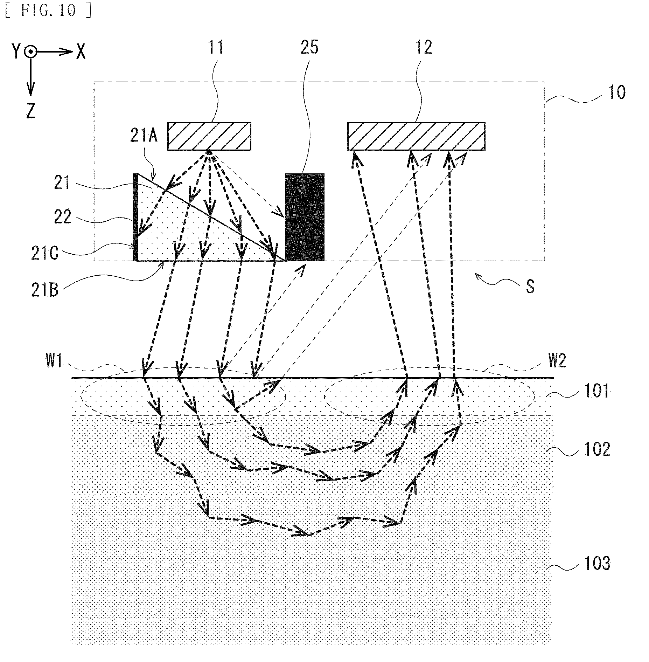

In the embodiment described above, the prism 23 is provided; however, this is non-limiting. Alternatively, for example, as illustrated in FIG. 10, the prism 23 may be omitted. Even in this case, the prism 21 so restricts the light traveling direction as to prevent the light emitted to the outside through the detection surface S from traveling, in the X direction, beyond the light blocking wall 25 into the direction in which the light receiving device 12 is provided. This prevents, for example, the light emitted to the outside through the surface 21B from easily entering the epidermis portion W2 below the light receiving device 12. This reduces the amount of the light propagating through the epidermis portion W2, of the epidermis 101, below the light receiving device 12, and thus makes it possible to suppress an increase in the DC component included in the light to be detected by the light receiving device 12. In addition, as with the case of the embodiment described above, the light blocking wall 25 allows for blocking, to some extent, of the light from the epidermis portion W1 below the light emitting device 11, which makes it possible to suppress an increase in the DC component included in the light to be detected by the light receiving device 12. As a result, the biological information detection apparatus 1 makes it possible to improve the detection accuracy.

[Modification Example 2]

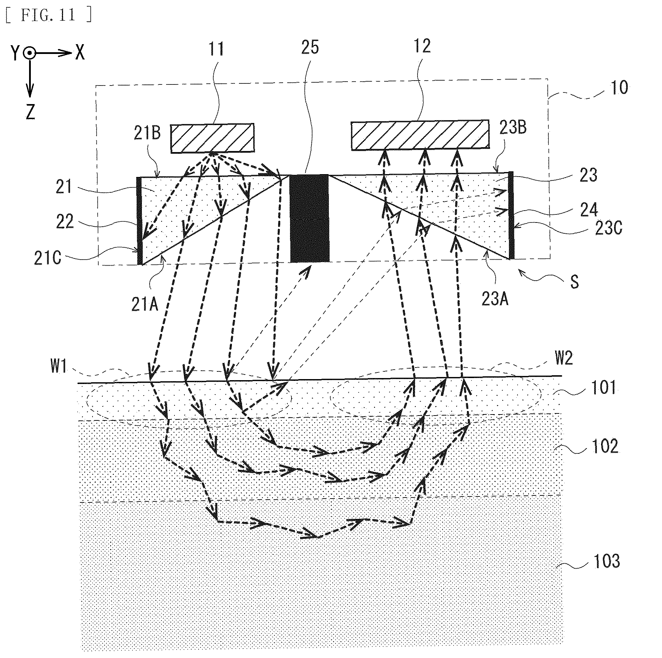

In the embodiment described above, the inclined surface 21A of the prism 21 faces the light emitting device 11, and the inclined surface 23A of the prism 23 faces the light receiving device 12; however, this is non-limiting. Alternatively, for example, as illustrated in FIG. 11, the inclined surface 21A of the prism 21 may face the detection surface S, and the inclined surface 23A of the prism 23 may face the detection surface S.

In this case, the prism 21 so restricts the light traveling direction as to prevent light emitted to the outside through the inclined surface 21A from traveling, in the X direction, beyond the light blocking wall 25 into the direction (a right direction in FIG. 11) in which the light receiving device 12 is provided, and so controls the light traveling direction as to cause the light emitted to the outside through the inclined surface 21A to travel while spreading, in the X direction, in the direction (a left direction in FIG. 11) opposite to the direction in which the light receiving device 12 is provided. In addition, the prism 21 so controls the light traveling direction as to cause the light emitted to the outside through the inclined surface 21A to travel while spreading in the Y direction.

In addition, the prism 23 so restricts the light traveling direction as to prevent light that has entered the inclined surface 23A from the direction in which the light emitting device 11 is provided and has an incident angle of the predetermined angle or greater, out of light entering the inclined surface 23A from the outside, from traveling, in the X direction, toward the light receiving device 12. In this example, the prism 23 so restricts the light traveling direction as to prevent the light that has entered the inclined surface 23A from traveling toward the light receiving device 12, by refracting the light that has entered the inclined surface 23A.

Note that, in the example in FIG. 11, the inclined surface 21A of the prism 21 faces the detection surface S, and the inclined surface 23A of the prism 23 faces the detection surface S; however, this is non-limiting. Alternatively, for example, either the inclined surface 21A of the prism 21 or the inclined surface 23A of the prism 23 may face the detection surface S.

[Modification Example 3]

In the embodiment described above, the prisms 21 and 23 are provided to suppress degradation of the detection accuracy in the case where the detection surface S of the biological information detection apparatus 1 is spaced away from the surface of the skin. In a case where the detection surface S of the biological information detection apparatus 1 can be further spaced away from the surface of the skin, it is possible to suppress degradation of the detection accuracy by various methods described below.

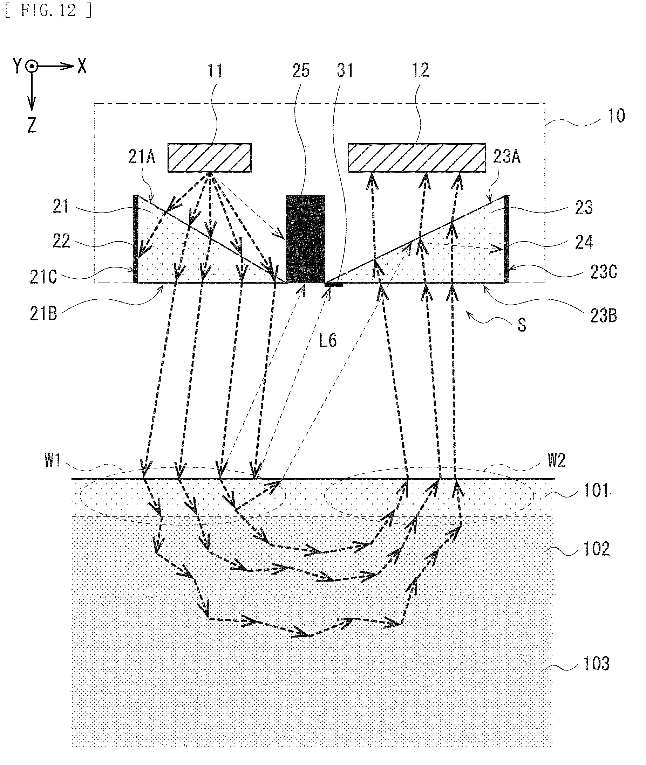

FIG. 12 illustrates an example of the biological information detection apparatus 1 according to the present modification example. The biological information detection apparatus 1 includes a light blocking film 31. The light blocking film 31 is provided in a region, on the surface 23B of the prism 23, in the vicinity of the light blocking wall 25. The light blocking film 31 blocks light (light L6) that travels from the epidermis portion W1 below the light emitting device 11 toward the end part, of the prism 23, on the light emitting device 11 side. Here, the light blocking film 31 corresponds to a specific example of a "second light blocking member" in one embodiment of the present disclosure.

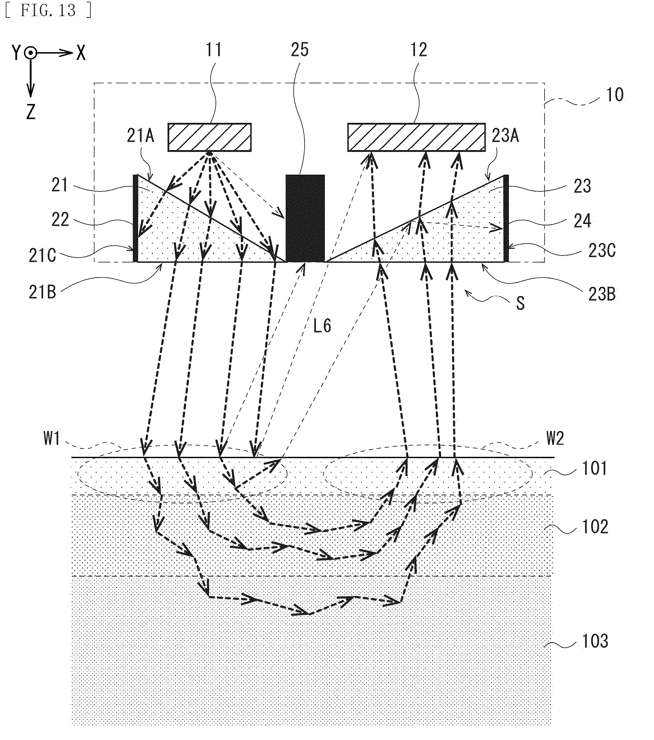

That is, in this example, as compared with the case in FIG. 5, the detection surface S of the biological information detection apparatus 1 is further spaced away from the surface of the skin. Therefore, an incident angle at which the light L6 enters the surface 23B of the prism 23 tends to be smaller than that in the case in FIG. 5. Accordingly, for example, in a case where the light blocking film 31 is not provided, as illustrated in FIG. 13, the light L6 may not be totally reflected at the inclined surface 23A of the prism 23 and can enter the light receiving device 12. In this case, the DC component included in the light to be detected by the light receiving device 12 increases, which results in degradation of the detection accuracy.

In contrast, in the biological information detection apparatus 1 according to the present modification example, the light L6 is blocked because the light blocking film 31 is provided. Accordingly, because the light L6 does not enter the light receiving device 12, the biological information detection apparatus 1 makes it possible to reduce the DC component included in the light to be detected by the light receiving device 12, and thus makes it possible to suppress degradation of the detection accuracy.

Note that although the light blocking film 31 is provided in this example, for example, the light blocking wall 25 may be increased in width in the X direction, without providing the light blocking film 31.

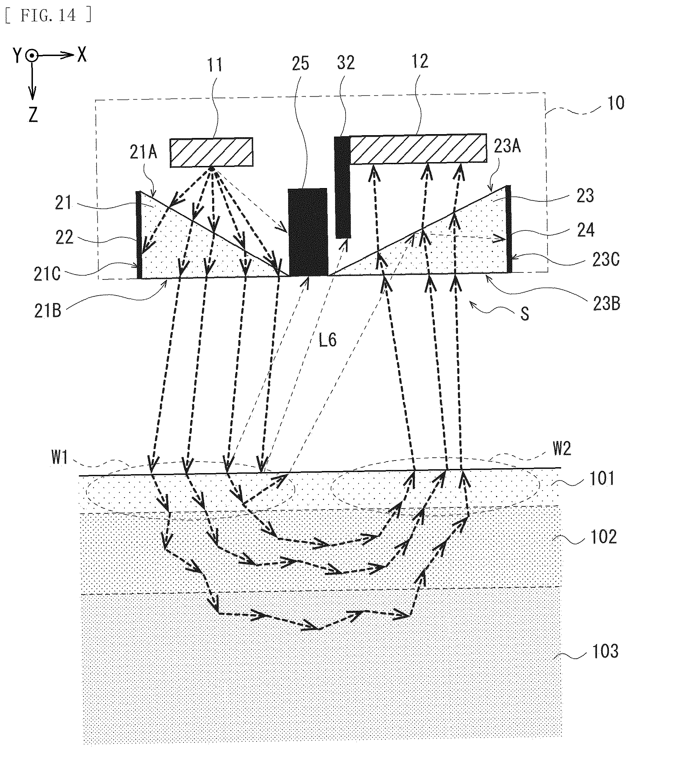

FIG. 14 illustrates another example of the biological information detection apparatus 1 according to the present modification example. The biological information detection apparatus 1 includes a light blocking wall 32. The light blocking wall 32 is provided between the light blocking wall 25 and the light receiving device 12 in the X direction. In addition, the light blocking wall 32 is provided to be spaced away from the detection surface S in the Y direction. The light blocking wall 32 blocks the light (the light L6) that travels from the epidermis portion W1 below the light emitting device 11 toward the end part, of the prism 23, on the light emitting device 11 side. Accordingly, because the light L6 does not enter the light receiving device 12, the biological information detection apparatus 1 makes it possible to reduce the DC component included in the light to be detected by the light receiving device 12, and thus makes it possible to suppress degradation of the detection accuracy. Here, the light blocking wall 32 corresponds to a specific example of a "third light blocking member" in one embodiment of the present disclosure.

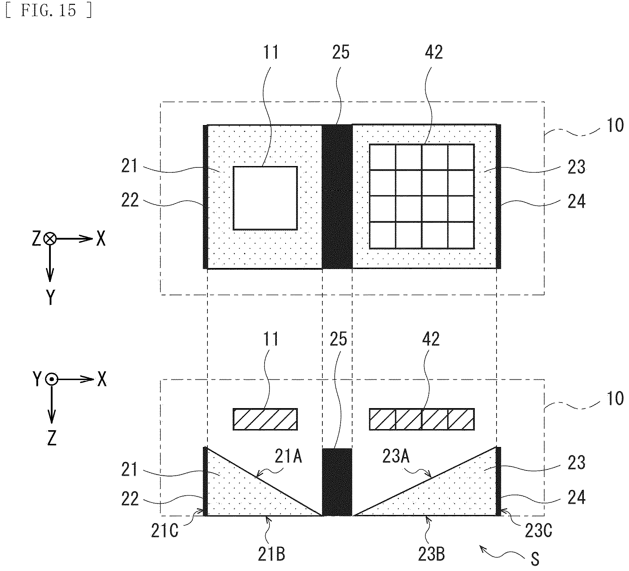

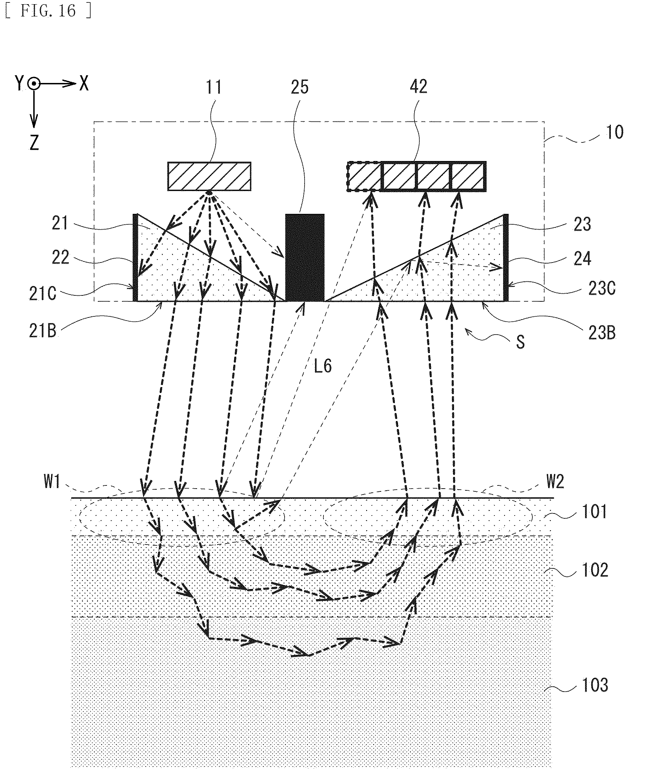

FIG. 15 illustrates another example of the biological information detection apparatus 1 according to the present modification example. FIG. 16 illustrates an operation example of the biological information detection apparatus 1. The biological information detection apparatus 1 includes a light receiving device 42. The light receiving device 42 includes sixteen (= 4 × 4) PDs arranged in a matrix. In FIG. 15, each of sixteen regions in the light receiving device 42 indicates the PD. The sixteen PDs in the light receiving device 42 are each configured to be, for example, individually caused to operate by the controller 14, or individually caused to stop the operation by the controller 14.

For example, as illustrated in FIG. 16, in the case where the detection surface S of the biological information detection apparatus 1 is spaced away from the surface of the skin, the light L6 is not totally reflected at the inclined surface 23A of the prism 23 and enters the light receiving device 12. In the case where the detection surface S of the biological information detection apparatus 1 is spaced away from the surface of the skin, the controller 14 stops respective operations of four PDs close to the light emitting device 11, out of the sixteen PDs in the light receiving device 42. In FIG. 16, a thick solid line indicates a part, in the light receiving device 42, corresponding to the PDs that operate, and a thick dashed line indicates a part, in the light receiving device 42, corresponding to the PDs that stop the respective operations. Accordingly, because the light receiving device 42 is prevented from detecting the light L6, the biological information detection apparatus 1 makes it possible to reduce the DC component included in light to be detected by the light receiving device 42, and to thus suppress degradation of the detection accuracy.

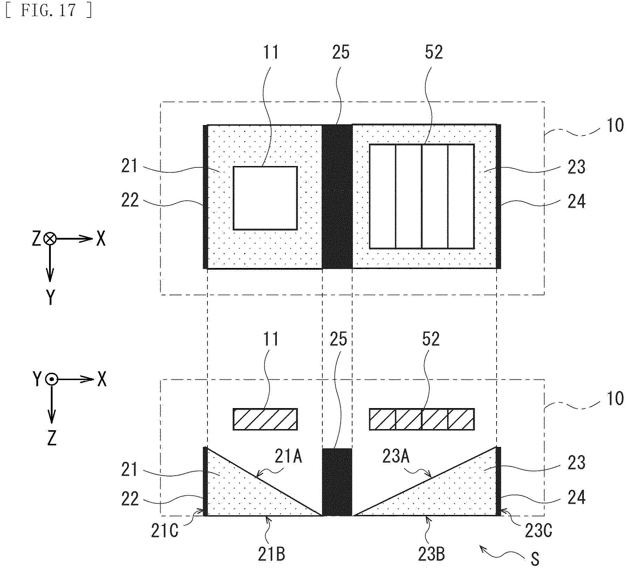

Note that in this example, the biological information detection apparatus 1 is provided with the light receiving device 42 including the plurality of PDs arranged in a matrix; however, this is non-limiting. Alternatively, for example, as in the biological information detection apparatus 1 illustrated in FIG. 17, a light receiving device 52 that is a line sensor may be provided. The light receiving device 52 includes four PDs each extending in the Y direction. The four PDs in the light receiving device 42 are each configured to be, for example, individually caused to operate by the controller 14, or individually caused to stop the operation by the controller 14. In the case where the detection surface S of the biological information detection apparatus 1 is spaced away from the surface of the skin, the controller 14 stops an operation of a PD close to the light emitting device 11, out of the four PDs in the light receiving device 52. Accordingly, because the light receiving device 52 is prevented from detecting the light L6, the biological information detection apparatus 1 makes it possible to reduce the DC component included in light to be detected by the light receiving device 52, and to thus suppress degradation of the detection accuracy.

[Modification Example 4]

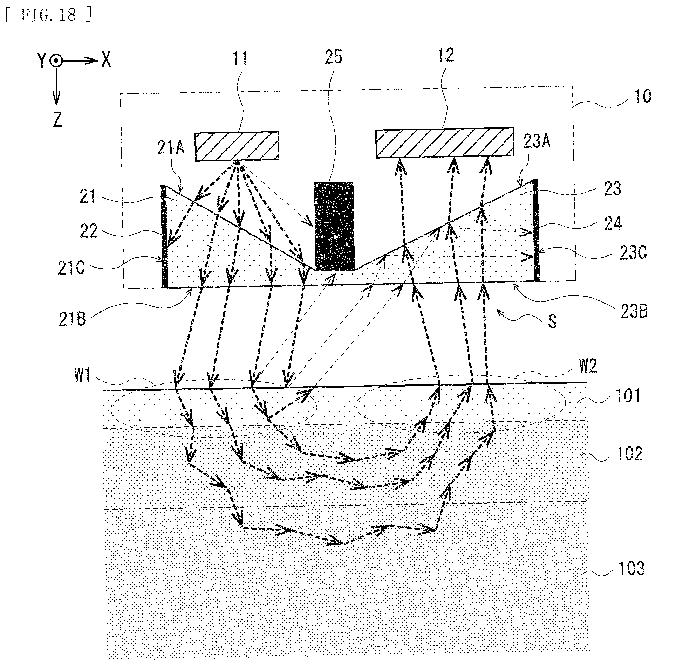

In the embodiment described above, the prisms 21 and 23 are provided as respective pieces separated from each other; however, this is non-limiting. Alternatively, for example, as illustrated in FIG. 18, the prisms 21 and 23 may be provided together as one piece. In this example, the prism 21 and the prism 23 are coupled to each other near the light blocking wall 25.

[Modification Example 5]

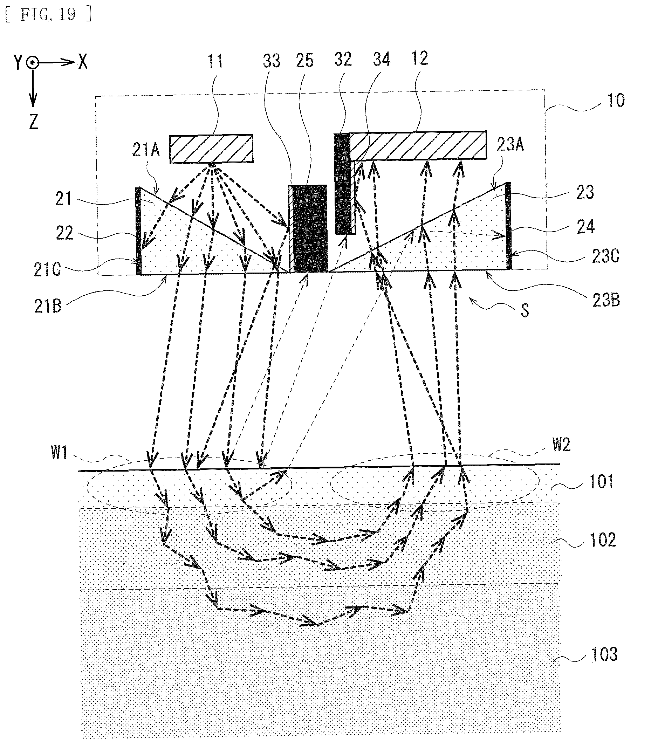

In the embodiment described above, for example, the light blocking wall 25 blocks the light directly arriving at the light blocking wall 25 from the light emitting device 11; however, this is non-limiting. Alternatively, for example, as illustrated in FIG. 19, a reflective film may be provided to reflect the light emitted from the light emitting device 11. The biological information detection apparatus 1 illustrated in FIG. 19 corresponds to the biological information detection apparatus 1 illustrated in FIG. 14 to which the present modification example is applied. The biological information detection apparatus 1 includes the light blocking wall 32 and reflective films 33 and 34.

The reflective film 33 is provided on a surface, of the light blocking wall 25, on a side where the prism 21 is provided, in the X direction. The reflective film 33 is configured to reflect, for example, the light from the light emitting device 11. Accordingly, the reflective film 33 reflects light that would be blocked by the light blocking wall 25 if it were not for the reflective film 33. This makes it possible to reduce light loss in the biological information detection apparatus 1. In addition, in the biological information detection apparatus 1, providing the reflective film 33 makes it possible to prevent the light emitted from the light emitting device 11 from entering the epidermis portion W2 below the light receiving device 12.

The reflective film 34 is provided on a surface, of the light blocking wall 32, on a side where the light receiving device 12 is provided, in the X direction. The reflective film 34 is configured to reflect, for example, light from the prism 23. This allows, in the biological information detection apparatus 1, the light from the epidermis portion W2 below the light receiving device 12 to easily enter the light receiving device 12.

Here, the reflective film 33 corresponds to a specific example of a "first reflective member" in one embodiment of the present disclosure. The reflective film 34 corresponds to a specific example of a "second reflective member" in one embodiment of the present disclosure.

In the above-described manner, the biological information detection apparatus 1 makes it possible to increase the AC component included in the light to be detected by the light receiving device 12, and to suppress an increase in the DC component. As a result, the biological information detection apparatus 1 makes it possible to efficiently detect a change in volume of blood in the living body, and to thus improve the detection accuracy.

Note that in this example, both the reflective film 33 and the reflective film 34 are provided; however, this is non-limiting. For example, only either of the reflective films 33 and 34 may be provided. Further, in this example, the present modification example is applied to the biological information detection apparatus 1 illustrated in FIG. 14; however, this is non-limiting. For example, the present modification example may be applied to the biological information detection apparatus 1 illustrated in FIG. 5.

[Modification Example 6]

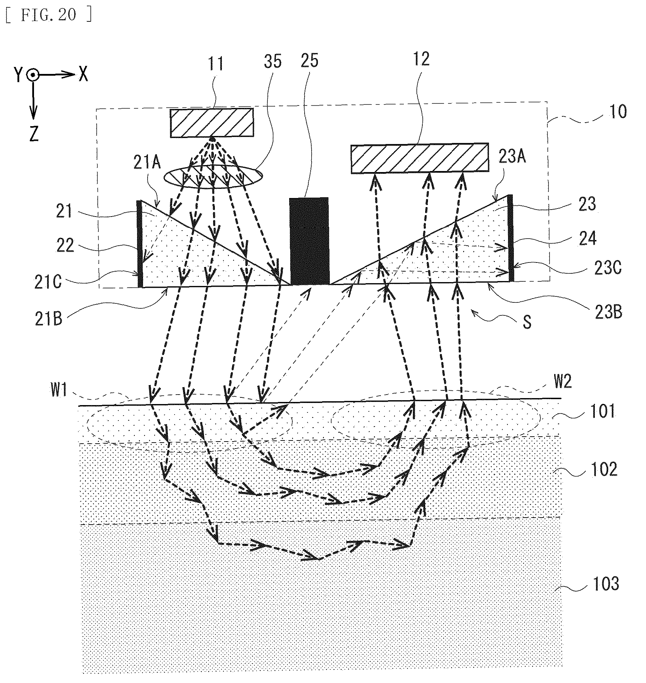

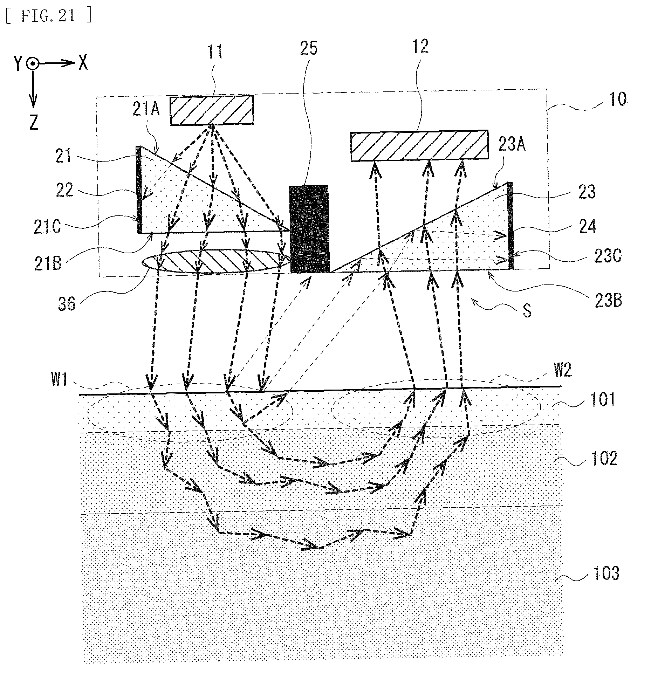

In the embodiment described above, the prism 21 is provided between the light emitting device 11 and the detection surface S; however, this is non-limiting. For example, as illustrated in FIGs. 20 and 21, another optical device may further be provided. The biological information detection apparatus 1 illustrated in FIG. 20 includes a lens 35. The lens 35 is provided between the light emitting device 11 and the prism 21. The biological information detection apparatus 1 illustrated in FIG. 21 includes a lens 36. The lens 36 is provided between the prism 21 and the detection surface S. Note that in the example in each of FIGs. 20 and 21, the lens is provided between the light emitting device 11 and the detection surface S; however, this is non-limiting. Alternatively, an optical device other than the lens may be provided.

Similarly, although the prism 23 is provided between the light receiving device 12 and the detection surface S in the embodiment described above, this is non-limiting, and another optical device may further be provided. Specifically, for example, an optical device such as a lens may be provided between the light receiving device 12 and the prism 23, or an optical device such as a lens may be provided between the prism 23 and the detection surface S.

[Modification Example 7]

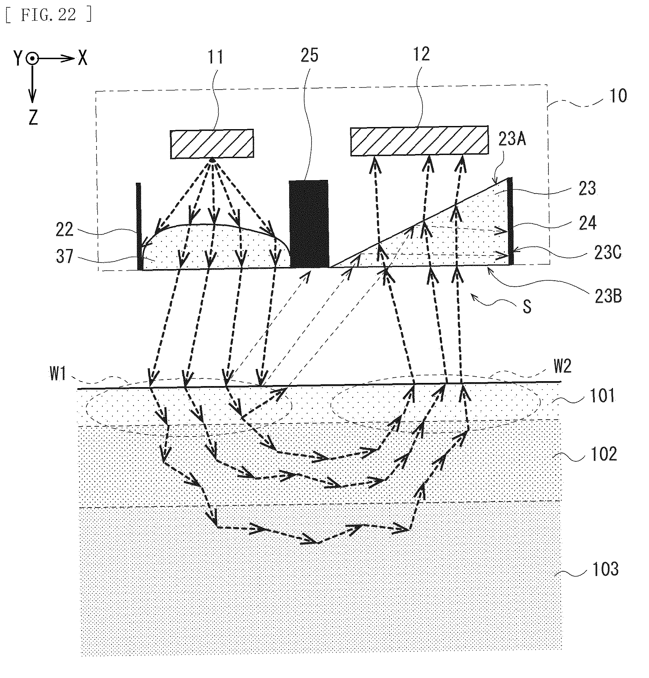

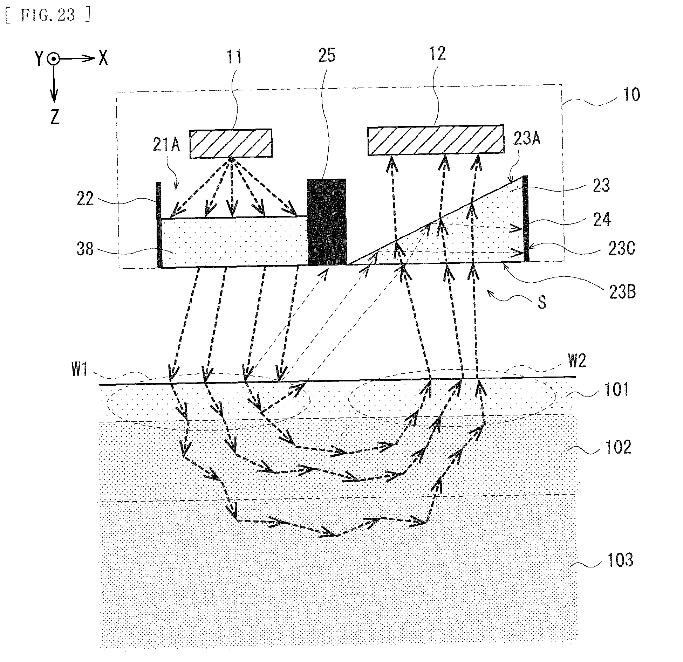

In the embodiment described above, the prism 21 is provided; however, this is non-limiting. For example, as illustrated in FIGs. 22 and 23, another optical device may be provided instead of the prism 21. The biological information detection apparatus 1 illustrated in FIG. 22 includes a lens 37. The lens 37 is provided between the light emitting device 11 and the detection surface S. The lens 37 is configured to control the traveling direction of the light from the light emitting device 11 to cause the light to travel toward the region WO illustrated in FIG. 6. The biological information detection apparatus 1 illustrated in FIG. 23 includes a diffraction grating 38. The diffraction grating 38 is provided between the light emitting device 11 and the detection surface S. The diffraction grating 38 is configured to control the traveling direction of the light from the light emitting device 11 to cause the light to travel toward the region WO illustrated in FIG. 6.

Similarly, although the prism 23 is provided in the embodiment described above, this is non-limiting, and another optical device such as a lens or a diffraction grating may be provided instead of the prism 23.

[Other Modification Examples]

In addition, two or more of the above-described modification examples may be combined with each other.

<2. Application Example>

Next, a description is given of an application example of the biological information detection apparatus described in each of the embodiment and the modification examples described above.

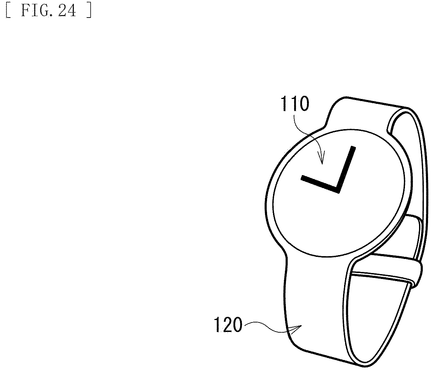

FIG. 24 illustrates an outer appearance of a watch to which the biological information detection apparatus according to the above-described embodiment or the like is to be applied. The watch includes, for example, a dial face 110 and a band part 120. A pulse measuring apparatus according to the above-described embodiment or the like is mounted on a back-side surface, of the dial face 110, that is to be in contact with a user's arm.

In addition to such a watch, the biological information detection apparatus according to the above-described embodiment or the like is applicable to various items to be worn by a user including, without limitation, a wristband, glasses, and a ring. It is thus possible to configure a wearable terminal configured to detect biological information such as a blood flow velocity or a blood volume pulse.

Although the present technology has been described with reference to the embodiment, some modification examples, and the example of application to electronic equipment, the present technology is not limited to the embodiment, etc., and various modifications may be made.

For example, in the above-described embodiment or the like, the light receiving device 12 includes one PD; however, this is non-limiting. Alternatively, for example, two or more PDs may be included. In addition, the light receiving device 12 may include, for example, an image sensor including a plurality of pixels.

Note that the effects described herein are merely examples and non-limiting, and any other effect may be achieved.

Note that the present technology may have any of the following configurations. According to the present technology having any of the following configurations, it is possible to improve detection accuracy.

(1) A biological information detection apparatus including:

a housing including a detection surface;
a light emitting device configured to emit light toward the detection surface;
a first optical member provided between the light emitting device and the detection surface of the housing;
a first light blocking member configured to block light; and
a light receiving device configured to detect light entering the detection surface, in which
the light emitting device, the first light blocking member, and the light receiving device are disposed in this order in a first direction that is along the detection surface, and
the first optical member is configured to restrict a light traveling direction to prevent light emitted to an outside through the detection surface from traveling, in the first direction, beyond the first light blocking member into a direction in which the light receiving device is provided, the first optical member being configured to control the light traveling direction to cause the light emitted to the outside through the detection surface to travel while spreading, in the first direction, in a direction opposite to the direction in which the light receiving device is provided.

(2) The biological information detection apparatus according to (1) described above, in which the first optical member is configured to control the light traveling direction to cause the light emitted to the outside through the detection surface to travel while spreading along the detection surface, in a second direction intersecting with the first direction.

(3) The biological information detection apparatus according to (1) or (2) described above, in which the first optical member includes a first inclined surface inclined in the first direction, and has a thickness that decreases, in the first direction, toward the direction in which the light receiving device is provided.

(4) The biological information detection apparatus according to (3) described above, in which the first inclined surface faces the light emitting device.

(5) The biological information detection apparatus according to (3) described above, in which the first inclined surface faces the detection surface.

(6) The biological information detection apparatus according to any one of (1) to (5) described above, further including a first reflective member provided on a surface, of the first light blocking member, on a side, in the first direction, where the first optical member is provided.

(7) The biological information detection apparatus according to any one of (1) to (6) described above, further including

a second optical member provided between the light receiving device and the detection surface of the housing, in which
the second optical member is configured to restrict the light traveling direction to prevent light that has entered the detection surface from a direction in which the light emitting device is provided and has an incident angle of a predetermined angle or greater, out of light entering the detection surface from the outside, from traveling, in the first direction, toward the light receiving device.

(8) The biological information detection apparatus according to (7) described above, in which the second optical member includes a second inclined surface inclined in the first direction, and has a thickness that decreases, in the first direction, toward the direction in which the light emitting device is provided.

(9) The biological information detection apparatus according to (8) described above, in which the second inclined surface faces the light receiving device.

(10) The biological information detection apparatus according to (8) described above, in which the second inclined surface faces the detection surface.

(11) The biological information detection apparatus according to (7) described above, in which the first optical member and the second optical member are configured together as one piece.

(12) The biological information detection apparatus according to any one of (7) to (11) described above, further including a second light blocking member provided in a region, on the detection surface, between the first light blocking member and the second optical member in the first direction.

(13) The biological information detection apparatus according to any one of (7) to (11) described above, further including a third light blocking member provided at a position spaced away from the detection surface, between the first light blocking member and the light receiving device in the first direction.

(14) The biological information detection apparatus according to (13) described above, further including a second reflective member provided on a surface, of the third light blocking member, on a side, in the first direction, where the light receiving device is provided.

(15) The biological information detection apparatus according to any one of (7) to (11) described above, further including

a controller configured to control an operation of the light receiving device, in which

the light receiving device includes a plurality of light receiving devices, and

the controller is configured to determine the light receiving device that is to be caused to operate, out of the plurality of light receiving devices.

The present application claims the benefit of Japanese Priority Patent Application JP2022-184026 filed with the Japan Patent Office on November 17, 2022, the entire contents of which are incorporated herein by reference.

It should be understood by those skilled in the art that various modifications, combinations, sub-combinations and alterations may occur depending on design requirements and other factors insofar as they are within the scope of the appended claims or the equivalents thereof.

## Claims

1.  A biological information detection apparatus comprising:

    a housing including a detection surface;
    a light emitting device configured to emit light toward the detection surface;
    a first optical member provided between the light emitting device and the detection surface of the housing;
    a first light blocking member configured to block light; and
    a light receiving device configured to detect light entering the detection surface, wherein
    the light emitting device, the first light blocking member, and the light receiving device are disposed in this order in a first direction that is along the detection surface, and
    the first optical member is configured to restrict a light traveling direction to prevent light emitted to an outside through the detection surface from traveling, in the first direction, beyond the first light blocking member into a direction in which the light receiving device is provided, the first optical member being configured to control the light traveling direction to cause the light emitted to the outside through the detection surface to travel while spreading, in the first direction, in a direction opposite to the direction in which the light receiving device is provided.

2.  The biological information detection apparatus according to claim 1, wherein the first optical member is configured to control the light traveling direction to cause the light emitted to the outside through the detection surface to travel while spreading along the detection surface, in a second direction intersecting with the first direction.

3.  The biological information detection apparatus according to claim 1, wherein the first optical member includes a first inclined surface inclined in the first direction, and has a thickness that decreases, in the first direction, toward the direction in which the light receiving device is provided.

4.  The biological information detection apparatus according to claim 3, wherein the first inclined surface faces the light emitting device.

5. The biological information detection apparatus according to claim 3, wherein the first inclined surface faces the detection surface.

6. The biological information detection apparatus according to claim 1, further comprising a first reflective member provided on a surface, of the first light blocking member, on a side, in the first direction, where the first optical member is provided.

7. The biological information detection apparatus according to claim 1, further comprising

   a second optical member provided between the light receiving device and the detection surface of the housing, wherein
   the second optical member is configured to restrict the light traveling direction to prevent light that has entered the detection surface from a direction in which the light emitting device is provided and has an incident angle of a predetermined angle or greater, out of light entering the detection surface from the outside, from traveling, in the first direction, toward the light receiving device.

8. The biological information detection apparatus according to claim 7, wherein the second optical member includes a second inclined surface inclined in the first direction, and has a thickness that decreases, in the first direction, toward the direction in which the light emitting device is provided.

9. The biological information detection apparatus according to claim 8, wherein the second inclined surface faces the light receiving device.

10. The biological information detection apparatus according to claim 8, wherein the second inclined surface faces the detection surface.

11. The biological information detection apparatus according to claim 7, wherein the first optical member and the second optical member are configured together as one piece.

12. The biological information detection apparatus according to claim 7, further comprising a second light blocking member provided in a region, on the detection surface, between the first light blocking member and the second optical member in the first direction.

13. The biological information detection apparatus according to claim 7, further comprising a third light blocking member provided at a position spaced away from the detection surface, between the first light blocking member and the light receiving device in the first direction.

14. The biological information detection apparatus according to claim 13, further comprising a second reflective member provided on a surface, of the third light blocking member, on a side, in the first direction, where the light receiving device is provided.

15. The biological information detection apparatus according to claim 7, further comprising

   a controller configured to control an operation of the light receiving device, wherein
   the light receiving device comprises a plurality of light receiving devices, and
   the controller is configured to determine the light receiving device that is to be caused to operate, out of the plurality of light receiving devices.

[ FIG. 1 ]

[ FIG. 2 ]

[ FIG. 3A ]

[ FIG. 3B ]

[ FIG. 4 ]

[ FIG. 5 ]

[ FIG. 6 ]

[ FIG. 7 ]

[ FIG. 8 ]

[ FIG. 9 ]

EP 4 620 385 A1

[ FIG. 10 ]

[ FIG. 11 ]

[ FIG. 12 ]

[ FIG. 13 ]

[ FIG. 14 ]

[ FIG. 15 ]

[ FIG. 16 ]

[ FIG. 17 ]

[ FIG. 18 ]

[ FIG. 19 ]

[ FIG.20 ]

[ FIG. 21 ]

[ FIG. 22 ]

[ FIG. 23 ]

[ FIG. 24 ]

110

120

## INTERNATIONAL SEARCH REPORT

| International application No. |
| --- |
| **PCT/JP2023/034798** |

| | |
| --- | --- |
| **A.** | **CLASSIFICATION OF SUBJECT MATTER** |

*A61B 5/02*(2006.01)i; *A61B 5/026*(2006.01)i
FI:   A61B5/02 310B; A61B5/026 120

According to International Patent Classification (IPC) or to both national classification and IPC

| | |
| --- | --- |
| **B.** | **FIELDS SEARCHED** |

Minimum documentation searched (classification system followed by classification symbols)

A61B5/02-5/03;

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

| | |
| --- | --- |
| **C.** | **DOCUMENTS CONSIDERED TO BE RELEVANT** |

| Category* | Citation of document, with indication, where appropriate, of the relevant passages | Relevant to claim No. |
| --- | --- | --- |
| A | JP 2022-156201 A (SEIKO EPSON CORP) 14 October 2022 (2022-10-14)<br>entire text, all drawings | 1-15 |
| A | JP 2017-169690 A (SONY CORP) 28 September 2017 (2017-09-28)<br>entire text, all drawings | 1-15 |
| A | US 2017/0164848 A1 (FITBIT, INC.) 15 June 2017 (2017-06-15)<br>entire text, all drawings | 1-15 |
| A | US 2018/0020960 A1 (BIOBEAT TECHNOLOGIES LTD) 25 January 2018 (2018-01-25)<br>entire text, all drawings | 1-15 |
| A | CN 113558594 A (HUAWEI TECHNOLOGIES CO., LTD.) 29 October 2021 (2021-10-29)<br>entire text, all drawings | 1-15 |

☐ Further documents are listed in the continuation of Box C.       ☑ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **29 November 2023** | **12 December 2023** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2023/034798**

| Patent document cited in search report | | | Publication date (day/month/year) | Patent family member(s) | | | Publication date (day/month/year) |
|---|---|---|---|---|---|---|---|
| JP | 2022-156201 | A | 14 October 2022 | US | 2022/0170852 | A1 | |
| | | | | CN | 114569099 | A | |
| JP | 2017-169690 | A | 28 September 2017 | (Family: none) | | | |
| US | 2017/0164848 | A1 | 15 June 2017 | CN | 106901703 | A | |
| US | 2018/0020960 | A1 | 25 January 2018 | WO | 2018/020492 | A1 | |
| | | | | EP | 3487394 | A1 | |
| | | | | CN | 109688905 | A | |
| CN | 113558594 | A | 29 October 2021 | US | 2023/0210389 | A1 | |
| | | | | WO | 2021/218861 | A1 | |
| | | | | EP | 4134001 | A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2020090883 A
- JP 2022184026 A